(19) Europäisches Patentamt
European Patent Office
Office européen des brevets

(11) Veröffentlichungsnummer : **0 105 393**
**B1**

(12) # EUROPÄISCHE PATENTSCHRIFT

(45) Veröffentlichungstag der Patentschrift : **05.03.86**

(51) Int. Cl.[4] : **C 07 H 19/06**, A 61 K 31/70, C 12 P 19/38

(21) Anmeldenummer : **83109229.1**

(22) Anmeldetag : **17.09.83**

(54) **Neue Nucleosidverbindungen, Verfahren zu ihrer Herstellung sowie ihre Verwendung als Arzneimittel.**

(30) Priorität : **01.10.82 DE 3236389**

(43) Veröffentlichungstag der Anmeldung : **18.04.84 Patentblatt 84/16**

(45) Bekanntmachung des Hinweises auf die Patenterteilung : **05.03.86 Patentblatt 86/10**

(84) Benannte Vertragsstaaten : **CH DE FR GB IT LI**

(56) Entgegenhaltungen :
**EP-A- 0 022 964**
**EP-A- 0 057 349**
**EP-A- 0 057 812**

(73) Patentinhaber : **BAYER AG**
**Konzernverwaltung RP Patentabteilung**
**D-5090 Leverkusen 1 Bayerwerk (DE)**

(72) Erfinder : **Benz, Günter, Dr.**
**Am Bölkumer Busch 5**
**D-5620 Velbert-Neviges 15 (DE)**
Erfinder : **Metzger, Karl Georg, Dr.**
**Pahlkestrasse 15**
**D-5600 Wuppertal 1 (DE)**
Erfinder : **Zeiler, Hans-Joachim, Dr.**
**Elsbekerstrasse 46**
**D-5620 Velbert-Neviges 15 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

**Beschreibung**

Aus den DE-OSsen 3 102 136 und 3 102 137 sind 4',6'-Didesoxy-4'-thio-6'-aminohepturonsäurenucleoside bekannt. Diese Verbindungen sind antibiotisch wirksam.

Die vorliegende Erfindung stellt Verbindungen der allgemeinen Formel 1 zur Verfügung,

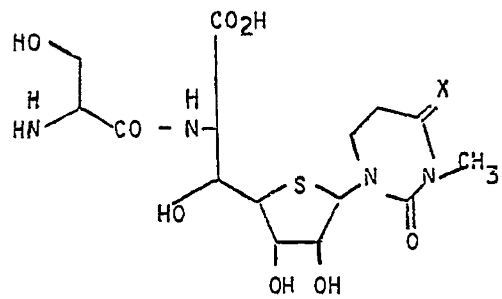

(1)

in der X = N—CO—$NH_2$ oder O sein kann.

Die erfindungsgemäßen Verbindungen können dadurch erhalten, werden, daß Verbindungen der Formel 2

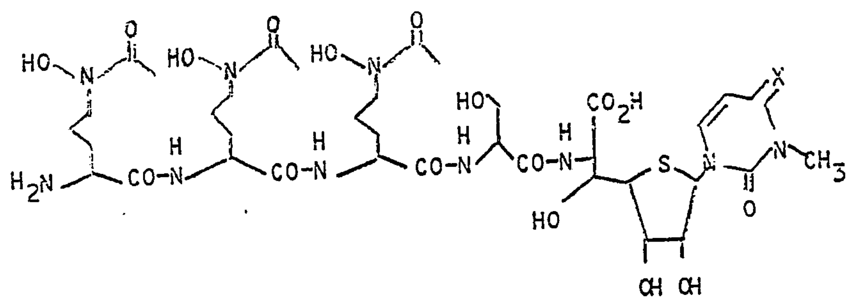

(2)

gespalten werden. Die Verbindungen der Formel 2 und ihre Herstellung sind in den eingangs erwähnten Offenlegungsschriften sowie in den zugehörigen EP-A 57 349 und 57 812 beschrieben.

Die Spaltung dieser Verbindungen zu den erfindungsgemäßen Verbindungen erfolgt vorzugsweise enzymatisch. Hierfür kommen vor allem Peptidhydrolasen der Gruppen EC 3.4.11, α-Aminoacyl-peptidhydrolasen und EC 3.4.21-23 Proteasen in Frage. Unter diesen sind besonders die Leucinaminopeptidase (Schweineniere, mikrosomal), α-Chymotrypsin, Subtilisin, Aminoacylase I, β-Lactamase aus E. coli T7, Proteinase K und Pronase E zu nennen.

Die jeweils optimalen Spaltbedindungen können durch einfache Versuche leicht ermittelt werden. Insbesondere die erforderlichen Reaktionszeiten ergeben sich dabei in Abhängigkeit von dem verwendeten Enzym. So läßt sich zum Beispiel die Spaltung mit der besonders bevorzugten Schweineniere-Leucinaminopeptidase bei 25 °C innerhalb von 40 h durchführen und sie gelingt beispielsweise mit der besonders bevorzugten mikrosomalen Leucinaminopeptidase (1 mmol Substrat, 29,3 U, U = eine Einheit hydrolysiert 1.0 µm von L-Leucinamid zu L-Leucin und $NH_3$ pro Minute bei pH 8.5 und 25 °C) innerhalb von 16 h bei pH 7.2 und 37 °C.

Ganz allgemein kann gesagt werden, daß die Reaktions-zeiten bei etwa 10-50 Stunden, vorzugsweise bei 15-40 Stunden und die Reaktionstemperatur bei etwa 20-40 °C liegt. Die Spaltung wird vorzugsweise in Gegenwart eines Puffers bei pH-Werten von 7-9 durchgeführt.

Im Anschluß an die Reaktion wird die jeweilige erfindungsgemäße Verbindung von etwa vorhandenen anderen Spaltprodukten abgetrennt, d. h. gereinigt.

Die Reinigung erfolgt dabei vorzugsweise durch Chromatographie. Vorzugsweise wird sie an Celluloseionenaustauschern (e. g. Sephadex® SP 25 H®, Natriumchloridgradient als Laufmittel), Adsorberharzen und an Kieselgel durchgeführt.

Die erfindungsgemäßen Verbindungen weisen überraschend antimikrobielle Wirksamkeit auf, die durch in vitro-Versuche demonstriert wird.

In vitro wird im Agar-Verdünnungstest nach dem international üblichen Test (American National Committee for Clinical Laboratory Standards = NCCLS) Wirksamkeit gegen nachfolgend aufgeführte Keime gefunden.

(Siehe Tabelle 1 Seite 3 f.)

Tabelle 1

| Verbindung | Keim | MHK (µg/ml) |
|---|---|---|
| 1 $X=NCONH_2$ | E.coli Neumann | 32 |
| | Klebs. 57 USA | 4 |
| | Staph.epid. 25185 | 64 |
| 1 X=O | Serratia 16001 | 128 |

Die Bestimmung der minimalen Hemmkonzentration (MHK) erfolgte mit einem Inoculator im Agarverdünnungsverfahren mit einer Einsaatdichte von $10^4$ pro Impf-Punkt. Die MHK ist die Konzentration, bei der keine Bakterienkolonien wachsen.

Die erfindungsgemäßen Verbindungen können somit zur Bekämpfung bakterieller Erkrankungen dienen, besonders in Form pharmazeutischer Zusammensetzungen. Solche pharmazeutischen Zubereitungen bestehen aus mindestens einem der erfindungsgemäßen Wirkstoffe und gegebenenfalls geeigneten nichttoxischen Träger- und Hilfsstoffen. Solche pharmazeutischen Zubereitungen gehören ebenfalls zur vorliegenden Erfindung.

Zur vorliegenden Erfindung gehören weiterhin pharmazeutische Zubereitungen in Dosierungseinheiten. Dies bedeutet, daß die Zubereitungen in Form einzelner Teile, z. B. Tabletten, Dragees, Kapseln, Pillen, Suppositorien und Ampullen vorliegen, deren Wirkstoffgehalt einem Bruchteil oder einem Vielfachen einer Einzeldosis entspricht. Die Dosierungseinheiten können z. B. 1, 2, 3, oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nichttoxischen, pharmazeutisch geeigneten Trägerstoffen sind feste, halbfeste oder flüssige Verdünnungsmittel, Füllstoffe und Formulierungshilfmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z. B. Stärken, Milchzucker, Rohrzucker, Glukose, Mannit und Kieselsäure, (b) Bindemittel, z. B. Carboxymethylcellulose, Alginate, Gelatine, Polyvinylpyrrolidon, (c) Feuchthaltemittel, z. B. Glycerin, (d) Sprengmittel, z. B. Agar-Agar, Calciumcarbonat und Natriumcarbonat, (e) Lösungsverzögerer, z. B. Paraffin und (f) Resorptionsbeschleuniger, z. B. quaternäre Ammoniumverbindungen, (g) Netzmittel, z. B. Cetylalkohol, Glycerinmonostearat, (h) Adsorptionsmittel, z. B. Kaolin und Bentonit und (i) Gleitmittel, z. B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen gegebenenfalls Opakisierungsmittel enthaltenden Überzügen und Hüllen versehen sein und auch so zusammengesetzt sein, daß sie den Wirkstoff nur oder bevorzugt in einem bestimmten Teil des Intestinaltraktes, gegebenenfalls verzögert abgeben, wobei als Einbettungsmassen z. B. Polymersubstanzen und Wachse verwendet werden können.

Der Wirkstoff kann gegebenenfalls mit einem oder mehreren der oben angegebenen Trägerstoffe auch in mikroverkapselter Form vorliegen.

Suppositorien können neben dem Wirkstoff die üblichen wasserlöslichen oder wasserunlöslichen Trägerstoffe enthalten, z. B. Polyethylenglykole, Fette, z. B. Kakaofett und höhere Ester (z. B. $C_{14}$-Alkohol mit $C_{16}$-Fettsäure) oder Gemische dieser Stoffe.

Salben, Pasten, Cremes und Gele können neben dem Wirkstoff die üblichen Trägerstoffe enthalten, z. B. tierische und pflanzliche Fette, Wachse, Paraffine, Stärke, Traganth, Cellulosederivate, Polyethylenglykol, Silicone, Bentonite, Kieselsäure, Talkum und Zinkoxid oder Gemische dieser Stoffe. Puder und Sprays können neben dem Wirkstoff die üblichen Trägerstoffe enthalten, z. B. Milchzucker, Talkum, Kieselsäure, Aluminiumhydroxid, Calciumsilikat und Polyamidpulver oder Gemische dieser Stoffe. Sprays können zusätzlich die üblichen Treibmittel, z. B. Chlorfluorkohlenwasserstoffe enthalten.

Lösungen und Emulsionen können neben dem Wirkstoff die üblichen Trägerstoffe wie Lösungsmittel, Lösungsvermittler und Emulgatoren, z. B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem Wirkstoff die üblichen Trägerstoffe wie flüssige Verdünnungsmittel, z. B. Wasser, Ethylalkohol, Propylenglykol, Suspendiermittel, z. B. ethoxylierte Isostearylalkohole, Polyoxyethylensorbit- und Sorbitanester, mikrokristalline Cellulose, Aluminiummetahydroxid, Bentonit, Agar-Agar und Traganth oder Gemische dieser Stoffe enthalten.

Die genannten Formulierungsformen können auch Färbemittel, Konservierungsmittel sowie geruchs- und geschmacksverbessernde Zusätze, z. B. Pfefferminzöl und Eukalyptusöl und Süßmittel, z. B. Saccharin enthalten.

Die erfindungsgemäßen Verbindungen sollen in den oben aufgeführten pharmezeutischen Zubereitungen vorzugsweise in einer Konzentration von etwa 0,1 bis 99,5, vorzugsweise von etwa 0,5 bis 95 Gewichtsprozent der Gesamtmischung vorhanden sein.

Die oben aufgeführten pharmazeutischen Zubereitungen können außer dem erfindungsgemäßen Wirkstoff auch weitere pharmazeutische Wirkstoffe enthalten.

Die Herstellung der oben aufgeführten pharmazeutischen Zubereitungen erfolgt in üblicher Weise nach bekannten Methoden, z. B. durch Mischen des Wirkstoffs mit dem oder den Trägerstoffen.

Zur vorliegenden Erfindung gehört auch die Verwendung der erfindungsgemäßen Wirkstoffe bei der Herstellung von Arzneimitteln die den erfindungsgemäßen Wirkstoff enthalten und die in der Human- und Veterinärmedizin bei der Verhütung, Besserung und/oder Heilung von Erkrankungen verwendet werden.

Der Wirkstoff oder die pharmazeutischen Zubereitungen können lokal, oral, parenteral, intraperitoneal und/oder rectal, vorzugsweise oral und parenteral, insbebesondere intramuskulär oder intravenös, gegebenenfalls auch als Dauertropfinfusion, appliziert werden.

Im allgemeinen empfiehlt es sich, die erfindungsgemäßen Wirkstoffe bei oraler oder parenteraler Applikation in Gesamtmengen von etwa 10 bis 1 000, vorzugsweise 50 bis 600 mg/kg Körpergewicht je 24 Stunden, gegebenenfalls in Form mehrerer Einzelgaben zur Erzielung der gewünschten Ergebnisse zu verabreichen. Eine Einzelgabe enthält die erfindungsgemäßen Wirkstoffe vorzugsweise in Mengen von etwa 50 bis etwa 300 mg/kg, insbesondere 100 bis 200 mg/kg Körpergewicht.

Es kann jedoch erforderlich sein, von den genannten Dosierungen abzuweichen und zwar in Abhängigkeit von der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Die erfindungsgemäßen Verbindungen können außerdem als Zwischenprodukte für die Herstellung anderer Verbindungen, z. B. anderer Wirkstoffe dienen. So ist es z. B. denkbar, wenn Derivate der Verbindungen gemäß den eingangs erwähnten Offenlegungsschriften gewünscht werden, dies aber wegen der Komplexität des Moleküls nur schwer erreichbar ist, die Derivatisierung statt dessen an den erfindungsgemäßen Verbindungen vorzunehmen und die so erhaltenen Derivate dann wieder mit dem abgespaltenen Rest zu rekombinieren.

## Beispiele

### Beispiel 1

Nucleosid 1 X = N—CO—$NH_2$

500 mg (0,5 mmol) der Verbindung gemäß Anspruch 1 der DE-OS 3 102 137 wurden in 250 ml bidest. Wasser gelöst und mit 0,5 n Trispuffer auf pH 8,5 eingestellt. Zu dieser Lösung wurden 0,35 ml (175 U) Leucinaminopeptidase (Schweineniere ; Böhringer 107 182 ; spez. Aktivität 100 U/mg, 25 °C Leucinamid als Substrat) gegeben und es wurde 40 h bei 25 °C gerührt. Nach Neutralisation mit 1 n HCl wurde der Ansatz gefriergetrocknet und an 50 ml Sephadex® SP 25 $H^{\oplus}$ chromatographiert (Vorlauf 300 ml bidest, Wasser, 900 ml Gradient 0,01 n-0,09 n NaCl-Lösung, Fluß 2 ml/min). In den Auslauf wurde 0,5 n Sörensenpuffer gepumpt, so daß die gesammelten Fraktionen pH 6,7 aufwiesen. Nach HPLC ($NH_2$-Säule 5 μm ; 4 × 250 mm Merck 50376, mobile Phase : Methanol/Zitronensäurepuffer, pH 4 ; 85 : 15 V/V ; Fließrate 2 ml/min) und DC-Analyse A : TLCOpti-RP $C_{12}$, Fluka, 1 n $(NH_4)_2CO_3$ ; B : TLC Kieselgel® 60 F 254, Merck, Acetonitril : $H_2O$ 8 : 2 ; C : TLC Kieselgel® 60 F 254, Merck, Zitronensäurepuffer, pH 5 : Acetonitril 5 % $FeCl_3/H_2O$ 85 : 15 : 0.1 wurden die identischen Fraktionen zusammengefaßt und an LGP 4067 (ein Lewatit®-Absorberharz der Bayer AG) entsalzt.

| | | | |
|---|---|---|---|
| Fr | 64 - 76 | 30,2 mg | |
| Fr | 77 - 130 | 37,8 mg | |
| Fr | 131 - 164 | 110,8 mg | 46,6 % |

$^1$H-NMR ($D_2O$, 250 MHz) : δ = 3.35 (s ; 3H, N—$CH_3$), 3.78 (dd, J = 6.0 Hz, J = 4.4 Hz ; 1H, H-4') ; 3.96 (dd, J = 12.2 Hz, J = 5.6 Hz ; 1H, O—CH Serin) ; 4.04 (dd, J = 12.2 Hz, J = 4.3 Hz, 1H, O—CH Serin) ; 4.21

(dd, J = 5.6 Hz, J = 4.3 Hz, 1H, N—CH Serin) ; 4.34-4.54 (m ; 4H, H-2', 3', 5',6') ; 5.94 (d, J = 5.3 Hz ; 1H, H-1'), 6.20 (d, J = 8.3 Hz ; 1H, H-5), 8.26 (d, J = 8.3 Hz, 1H, H-6).

UV (qualitativ 0,1 n HCl) $\lambda_{max}$ = 304 nm

Beispiel 2

Nucleosid 1 X = O

In zwei Parallelansätzen wurden jeweils 1,5 g (1,6 mmol) der Verbindung gemäß Anspruch 1 der DE-OS 3 102 136 in 500 ml bidest. Wasser gelöst und mit 0,5 n Trispuffer auf pH 7,2 eingestellt. Den Lösungen wurden jeweils 0,75 ml (46,9 U) Leucinaminopeptidase (mikrosomal ; Sigma L 5006, spez. Aktivität 10-20 U/mg, 37 °C, Leucinamid als Substrat) zugegeben und es wurde 16 h bei 37 °C gerührt. Die Reaktionslösungen wurden vereinigt und gefriergetrocknet.

Die Rohsubstanz wurde an 600 ml LGP 4067 entsalzt. Mit bidest. Wasser wurde solange gewaschen bis UV-aktives Material eluiert wurde. Das restliche Produkt wurde mit Methanol/Wasser 1 : 1 eluiert.

| | | | |
|---|---|---|---|
| Fr | 17- 45 | 3.2 g | Salz |
| Fr | 71- 74 | 0.2115 g | |
| Fr | 75-120 | 1.2342 g | 90.2 % |

H-NMR ($D_2O$, 250 MHz) : δ = 3.28 (s ; 3H, N—$CH_3$) ; 3.96 (dd, J = 12,5 Hz, J = 6.0 Hz ; O—CH Serin), 4.03 (dd, J = 12.5 Hz, J = 5.0 Hz, 1H, O—CH-Serin) ; 4.20 (t, J = 5 Hz ; 1H, N—CH-Serin) ; 4.43-4.51 (m ; 3H, H-2', 5') ; 4.55 (d, J = 5.5 Hz, 1H, H-6'), 5.93 (d, J = 5.0 Hz, 1H, H-1'), 5.96 (d, J = 8,0 Hz ; 1H, H-5) ; 8.45 (d, J = 8 Hz ; 1H, H-6).

UV (qualitativ, 0.1 n HCl) $\lambda_{max}$ = 264 nm.

**Patentansprüche**

1. Verbindungen der allgemeinen Formel 1

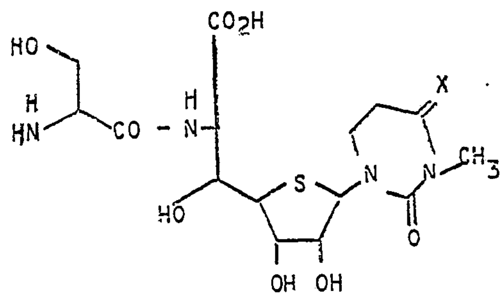

(1)

in der X = N—CO—$NH_2$ oder O bedeutet.

2. Verfahren zur Herstellung von Verbindungen der allgemeinen Formel 1

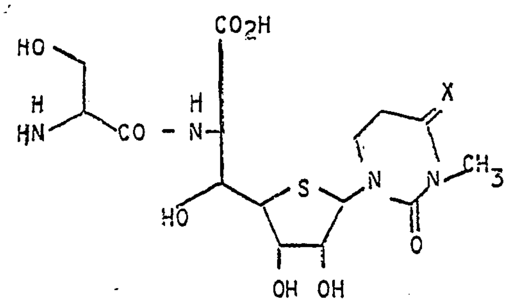

(1)

dadurch gekennzeichnet, daß man Verbindungen der Formel 2

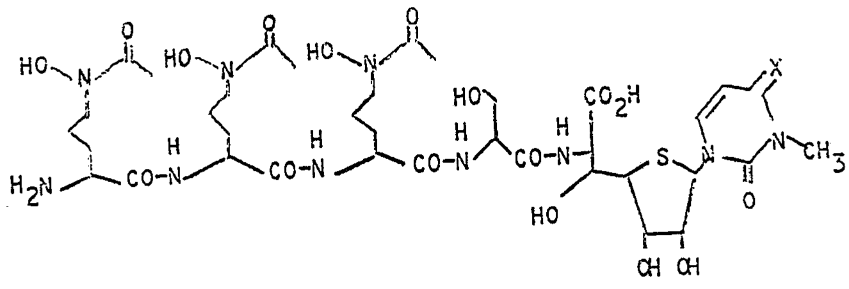

(2)

spaltet.

3. Verfahren nach Anspruch 2, dadurch gekennzeichnet, daß die Spaltung enzymatisch durchgeführt wird.

4. Verfahren nach Anspruch 3, dadurch gekennzeichnet, daß als Enzym Aminopeptidase verwendet wird.

5. Arzneimittel mit einem Gehalt an den Verbindungen gemäß Anspruch 1.

6. Verwendung der Verbindungen gemäß Anspruch 1 bei der Herstellung von Arzneimitteln.

**Claims**

1. Compounds of the general formula 1

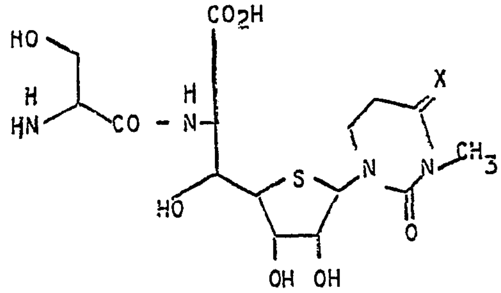

(1)

in which X denotes N—CO—$NH_2$ or O.

2. Process for the preparation of compounds of the general formula 1

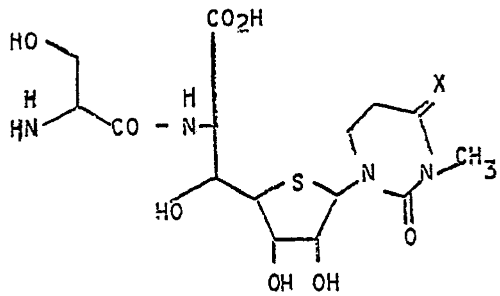

(1)

characterised in that compounds of the formula 2

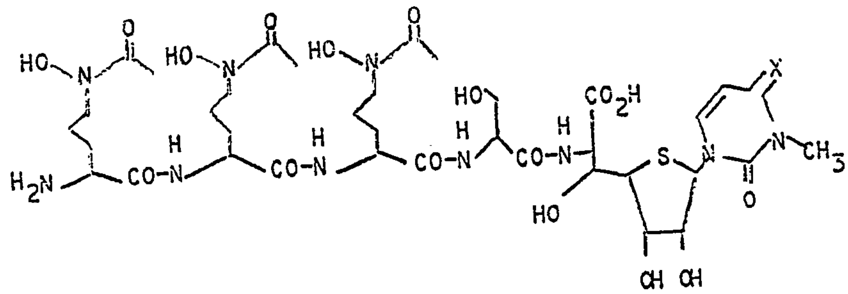

(2)

are cleaved.

3. Process according to Claim 2, characterised in that the cleavage is carried out enzymatically.

4. Process according to Claim 3, characterised in that aminopeptidase is used as the enzyme.

5. Medicaments containing the compounds according to Claim 1.

6. Use of the compounds according to Claim 1 in the production of medicaments.

**Revendications**

1. Composés de formule générale 1

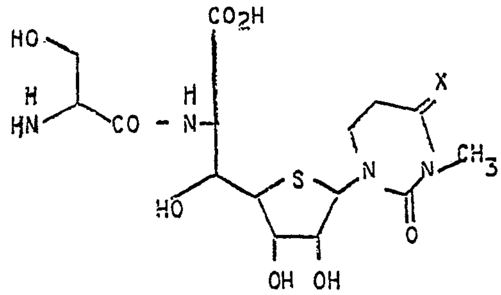

(1)

dans laquelle X représente N—CO—$NH_2$ ou un atome d'oxygène.

2. Procédé pour la fabrication des composés de formule générale 1

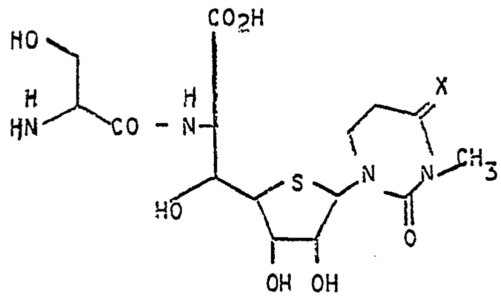

(1)

caractérisé en ce que l'on dissocie des composés de formule 2

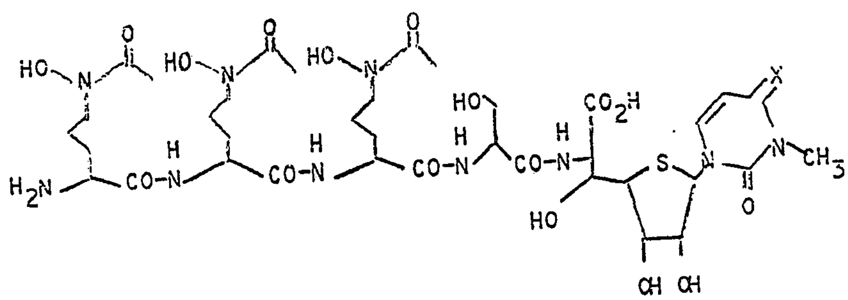

(2)

3. Procédé selon la revendication 2, caractérisé en ce que la dissociation est mise en œuvre par voie enzymatique.

4. Procédé selon la revendication 3, caractérisé en ce que l'on utilise comme enzyme une aminopeptidase.

5. Médicaments, caractérisés en ce qu'ils comprennent des composés selon la revendication 1.

6. Utilisation des composés selon la revendication 1 dans la fabrication de médicaments.